# FASCICULE DE BREVET EUROPEEN

(11) **EP 3 478 330 B1**
(45) Date de publication et mention de la délivrance du brevet: **29.07.2026**
(21) Numéro de dépôt: 17737237.2
(22) Date de dépôt: 29.06.2017
(51) Int. Cl.: A61L 12/08, C11D 3/386, C11D 3/48

(54) **PROCÉDÉ D'ÉLIMINATION DE BIOFILMS ET UTILISATION D'UNE COMPOSITION COMPRENANT UN COMPOSANT DÉTERGENT ET UN COMPOSANT ENZYMATIQUE**
VERFAHREN ZUR ENTFERNUNG VON BIOFILMEN UND VERWENDUNG EINER ZUSAMMENSETZUNG MIT EINER REINIGUNGSMITTELKOMPONENTE UND EINER ENZYMKOMPONENTE
PROCESS FOR REMOVING BIOFILMS AND USE OF A COMPOSITION COMPRISING A DETERGENT COMPONENT AND AN ENZYMATIC COMPONENT

(30) Priorité: 29.06.2016 BE 201605498
(43) Date de publication de la demande: 08.05.2019
(73) Titulaire: Realco, 1348 Louvain-la-Neuve (BE)
(72) Inventeur: BLACKMAN, Gordon, 1380 Lasnes (BE); FASTREZ, Sébastien, 7000 Mons (BE); GATHY, Camille, 1341 Céroux-Mousty (BE); BEBRONE, Carine, 4802 Heusy (BE); VANDEGAART, Hélène, 4654 Charneux (BE); DUYSENS, Guérin, 4121 Neuville-en-Condroz (BE)
(74) Mandataire: Calysta NV
(86) Numéro de dépôt international: PCT/EP2017/066081
(87) Numéro de publication internationale: WO 2018/002194

(56) Documents cités:
- EP-A1- 0 425 019
- EP-A1- 2 243 821
- EP-A1- 3 088 504
- EP-A2- 0 425 017
- EP-A2- 0 425 018
- WO-A1-2012/048757
- WO-A1-2012/048758
- WO-A1-98/50512
- WO-A2-2008/157350
- US-B1- 6 465 410

## Description

La présente invention se rapporte à un procédé d'élimination de biofilms et à l'utilisation d'une composition comprenant au moins un composant enzymatique et au moins un composant détergent.

Une telle composition est connue du document EP2243821 qui divulgue une composition pour l'élimination des biofilms présents sur un substrat, cette composition comprenant (1) un composant détergent contenant simultanément un agent mouillant, un agent séquestrant et un agent dispersant et (2) comprenant un composant enzymatique contenant simultanément au moins une protéase, au moins une laccase et au moins une polysaccharidase. Une telle composition est décrite comme pouvant être utilisée afin d'éliminer des biofilms dans des installations, pour le nettoyage de sols ou de surfaces en place ou par trempage, pour le nettoyage de matériel chirurgical en place ou par trempage et pour le nettoyage dans l'industrie alimentaire.

Le document WO2012/048757 A1 divulgue aussi une composition pour l'élimination des biofilms comprenant au moins un composant détergent et au moins un composant enzymatique.

Un biofilm est une pellicule visqueuse qui se développe sur toutes les surfaces, suite à l'adhésion de microorganismes sur ces surfaces et à la sécrétion par ceux-ci de polymères qui les recouvrent et facilitent leur adhésion. Les biofilms constituent ainsi une couche de protection autour des microorganismes et représentent une source récurrente de contamination du milieu environnant qui pose des problèmes, par exemple dans les secteurs de l'agro-alimentaire et dans les milieux hospitaliers.

Plus spécifiquement, l'accumulation de polymères sécrétés par les bactéries crée une matrice composée essentiellement de polysaccharides, d'ADN, de protéines ainsi que de lipides qui protège ces microorganismes des agressions extérieures et qui présente une très forte résistance aux procédures de nettoyage et de désinfection conventionnelles. Les microorganismes se développent donc aisément au sein de cette matrice protectrice et contaminent le milieu environnant en constituant un réservoir particulièrement critique et difficile à éliminer.

Il est reconnu que la problématique de la présence de biofilms est double. Premièrement, comme indiqué plus haut, ceux-ci représentent une source de contamination permanente et très difficile à éliminer par des moyens conventionnels, même les plus agressifs. En effet, les désinfectants classiques sont très souvent inefficaces car il est observé qu'ils ne parviennent pas à atteindre les microorganismes qui sont protégés par la matrice du biofilm composée de polysaccharides, d'ADN, de protéines et de lipides.

Deuxièmement, un biofilm est généralement mixte, en ce sens qu'il est initialement développé par certaines souches bactériennes mais qu'il peut en abriter d'autres, ces souches vivant et se développant en colonies. Or, ces colonies favorisent la communication entre bactéries et, entre autre, l'échange et la propagation de gènes de résistance portés par certaines bactéries. Les biofilms résultant de ces échanges de gènes sont alors encore plus difficiles à éliminer et il faut recourir à des moyens de désinfection ou de traitement de plus en plus puissants qui se heurtent toutefois fréquemment à des problèmes de résistance et/ou de tolérance majeurs.

La matrice de protection des bactéries formant les biofilms est si résistante qu'elle constitue une véritable barrière protégeant les bactéries de phénomènes de déshydratation, de l'action des antibiotiques et des biocides (et plus généralement des molécules microbicides), de la phagocytose et des acides.

En milieu hospitalier et vétérinaire mais aussi en milieu agro-alimentaire, la situation est d'autant plus critique que de nombreux microorganismes responsables de la formation de biofilms sont détectés en de nombreux endroits. Dans les hôpitaux et les cabinets vétérinaires, des biofilms sont détectés tant au niveau des individus (patients et animaux) qu'au niveau de l'environnement (salle d'opération, matériel chirurgical, équipements de maintenance de ce matériel, endoscopes, sondes urinaires, cathéters, équipement médical, appareil de dialyse ou de ventilation assistée des individus, ...) et des surfaces (sols, murs, tables d'opération, ...). Dans le secteur de l'agro-alimentaire et notamment dans les usines de fabrication de produits alimentaires, les biofilms peuvent être retrouvés au niveau des machines, des tables, des emballages et des opérateurs même si ces derniers prennent toutes les précautions possibles afin de ne pas contaminer les surfaces et les outils de travail.

De tout ceci, il ressort que les biofilms constituent une réelle problématique, tout particulièrement dans le domaine des soins de santé (hôpitaux, cabinets dentaires...), des soins vétérinaires et de l'agro-alimentaire. Cette problématique est d'autant plus critique que les biofilms peuvent impliquer des bactéries responsables d'infections potentiellement mortelles chez les individus que ces bactéries soient présentes dans les hôpitaux, les cabinets vétérinaires ou encore dans les produits alimentaires. Il convient donc de prendre toutes les précautions possibles afin d'éviter la formation et le développement de biofilms.

De nos jours, afin de lutter contre les biofilms, sont mises en œuvre des formulations enzymatiques comprenant une base détergente, ce que propose d'ailleurs le document EP2243821. La majorité des formulations actuelles repose plus particulièrement sur une association de plusieurs enzymes, ces formulations comprenant toujours généralement au moins une protéase. Toutefois, même les formulations (compositions) associant une base détergente et plusieurs enzymes présentent certaines limites en termes d'élimination des biofilms au départ d'un substrat (d'une surface).

Malheureusement, il s'avère donc actuellement que, même si une composition telle que celle divulguée dans le document EP2243821 se révèle être efficace en termes d'élimination de biofilms, les compositions d'élimination des biofilms reposent très souvent sur la mise en œuvre d'un cocktail enzymatique. Plus spécifiquement, en ce qui concerne le document EP2243821, la composition d'élimination de biofilms divulguée repose sur la mise en œuvre d'au moins trois enzymes (protéase et laccase et polysaccharidase) formulées dans une base détergente elle-même mettant en œuvre trois agents détergents différents (séquestrant et mouillant et dispersant). Il convient donc, pour formuler une composition d'élimination des biofilms selon le document EP2243821, de disposer d'au moins six composants différents, ce qui est économiquement peu avantageux dès lors qu'une manipulation de différents composants à formuler ensemble requiert du temps et qu'il faut se procurer ces six composants en parallèle.

Par ailleurs, il est reconnu que, dans une formulation comprenant plusieurs enzymes dont une protéase, cette dernière va inévitablement digérer graduellement au cours temps les autres enzymes présentes et forcément en réduire l'activité enzymatique au cours du temps, ceci ayant donc un impact sur la stabilité et l'efficacité des formulations comprenant une protéase. En outre, il s'avère que les compositions actuelles présentent certaines limites, notamment lorsqu'il s'agit d'éliminer de « vieux » biofilms, c'est-à-dire des biofilms en place depuis des mois voire des années.

D'autre part, même si ce n'est pas son objet, WO2008/157350 renseigne incidemment que la Dispersine B peut couper les liens b-1,6 du poly-N-acétyle glucosamine qui seraient présents dans un biofilm.

L'invention a pour but de pallier les inconvénients de l'état de la technique en procurant un procédé d'élimination de biofilms avec une composition qui est plus facile à formuler (à mettre en œuvre), dont la stabilité et l'efficacité au cours du temps est conservée et qui permet d'agir efficacement contre tout type de biofilms, ceci incluant les « vieux » biofilms.

Pour résoudre au moins en partie les problèmes de l'état de la technique, il est prévu suivant l'invention, l'utilisation d'une composition pour l'élimination des biofilms par nettoyage en place, par trempage ou par vaporisation de ladite surface, caractérisée en ce que ledit au moins un composant enzymatique comprend la β-1,6-N-acétylglucosaminidase et en ce que ledit composant détergent contient au moins un agent mouillant, au moins un agent séquestrant et au moins un agent dispersant, ledit agent mouillant représentant entre 1 et 30% du composant détergent, ledit agent séquestrant représentant entre 1 et 10% du composant détergent et ledit agent dispersant représentant entre 1 et 10% du composant détergent.

Au sens de la présente invention, le composant enzymatique peut comprendre la β-1,6-N-acétylglucosaminidase comme seule enzyme ou cette même enzyme associée à d'autres.

En particulier, la β-1,6-N-acétylglucosaminidase clive le polymère poly-β-1,6-N-acétyl-D-glucosamide (PNAG) qui est un exopolysaccharide.

De façon tout à fait surprenante et contre toute attente, il a été mis en évidence, dans le cadre de la présente invention, qu'une telle composition comprenant au moins la β-N-acétylglucosaminidase dans au moins un composant détergent qui comprend au moins un agent mouillant et au moins un agent séquestrant et au moins un agent dispersant est au moins aussi efficace pour l'élimination d'un biofilm qu'une composition selon le document EP2243821 alors que, comme indiqué plus haut, cette composition de l'état de la technique repose sur l'utilisation et la mise en œuvre conjointes de trois enzymes dans un même composant détergent. Il en découle que, selon la présente invention, une composition tout à fait efficace en termes d'élimination de biofilms sur une surface peut être préparée en ne mettant en œuvre que la β-1,6-N-acétylglucosaminidase au lieu d'un cocktail enzymatique reposant sur la présence simultanée de trois enzymes différentes. La formulation d'une telle composition est par conséquent plus aisée puisqu'elle doit mettre en oeuvre moins de composés différents, ce qui est économiquement plus rentable.

De façon encore plus surprenante et inattendue, il a été déterminé, dans le cadre de la présente invention, que les trois agents du composant détergent présentent un effet synergique optimisant l'efficacité de la β-1,6-N-acétylglucosaminidase en termes d'élimination du biofilm mais aussi en termes d'activité enzymatique de cette dernière au cours du temps. D'une part, il a en effet été mis en évidence que cette association particulière de trois agents détergents (au moins un agent mouillant + au moins un agent séquestrant + au moins un agent dispersant) assure une activité enzymatique significativement plus élevée de la β-1,6-N-acétylglucosaminidase au cours du temps par rapport aux activités enzymatiques relevées pour cette même enzyme lorsque le composant détergent ne comprend par exemple que deux de ces trois agents détergent. D'autre part, il a été mis en avant que cette association particulière de trois agents détergent augmente l'efficacité de la composition: une élimination optimale des biofilms est observée lorsque la β-1,6-N-acétylglucosaminidase est formulée dans un composant détergent comprenant simultanément au moins un agent mouillant, au moins un agent séquestrant et au moins un agent dispersant plutôt que dans un composant détergent ne comprenant par exemple que deux de ces trois agents détergent.

En outre, avec une telle composition et contrairement à une composition selon le document EP2243821, la présence d'une protéase n'est pas indispensablement requise afin d'obtenir une élimination adéquate des biofilms sur des surfaces, ceci permettant d'éliminer la problématique explicitée ci-dessus et liée à la présence de cette enzyme dans une formulation où sont présentes plusieurs enzymes.

Par ailleurs, au contraire des compositions d'élimination des biofilms connues de l'état de la technique, il a été mis en évidence qu'une composition comprenant au moins la β-1,6-N-acétylglucosaminidase dans au moins un composant détergent contenant au moins un agent mouillant et au moins un agent séquestrant et au moins un agent dispersant, permet d'agir efficacement même sur de « vieux » biofilms particulièrement difficiles à éliminer.

Dans le cadre de la présente invention, il a été montré que, suite à une éventuelle détection de la présence de biofilms, par exemple à l'aide d'un kit de détection tel que décrit dans le document EP2537601, un traitement avec une composition selon l'invention permet à la β-1,6-N-acétylglucosaminidase de dégrader efficacement et de manière polyvalente les polymères organiques de différentes natures constituant la matrice des biofilms formés par une multitude de microorganismes différents.

Sous l'action de la β-1,6-N-acétylglucosaminidase et conjointement à l'action du composant détergent, la matrice du biofilm est fragilisée et gonflée, ce qui permet de l'éliminer de la surface traitée. Par ailleurs, de façon surprenante, il a également été montré que l'utilisation de la composition selon l'invention n'est pas spécifique à un microorganisme particulier et donc à un type de biofilms particulier mais qu'elle est adaptée à de nombreuses souches bactériennes.

L'action détergente de la composition permet en outre d'assurer l'efficacité de la composition mise en œuvre dans le procédé selon l'invention. A cette fin, il est prévu suivant l'invention une base détergente (composant détergent) qui soit compatible et qui puisse agir de manière synergique avec l'activité enzymatique du composant enzymatique. En outre, suivant l'invention, il est prévu une base détergente qui permette d'améliorer significativement la rapidité et l'efficacité de l'élimination du biofilm. C'est pour ces raisons que la présente invention associe un agent mouillant et un agent dispersant et un agent séquestrant dans les proportions décrites ci-dessus. Les actions conjointes de ces trois agents du composant détergent de la composition permettent d'éliminer la partie superficielle du biofilm, de mouiller et de gonfler les structures organiques du biofilm favorisant donc de cette façon l'accessibilité du composant enzymatique qui fragilise et dégrade à son tour la matrice du biofilm.

Au sens de la présente invention, le composant détergent comprend au moins un agent mouillant et au moins un agent dispersant et au moins un agent séquestrant dans les proportions décrites ci-dessus. Comme indiqué plus haut, ce composant détergent agit tout d'abord en éliminant une partie superficielle du biofilm, ceci en mouillant et/ou en gonflant les structures organiques du biofilm. De cette façon, le composant détergent favorise l'accessibilité du composant enzymatique en déstructurant la matrice du biofilm. Le composant enzymatique agit ensuite en synergie avec le composant détergent et fragilise et dégrade à son tour la matrice du biofilm. Cette action combinée du composant enzymatique et du composant détergent, parfaitement compatible avec une action correcte de la β-1,6-N-acétylglucosaminidase, favorise l'accessibilité de la composition à des couches plus profondes des biofilms et permet un détachement rapide et optimal de tout type de biofilm tout en préservant le substrat traité (la surface traitée). Ainsi, le composant détergent permet aux enzymes d'agir rapidement sur l'ensemble des structures des biofilms.

L'agent dispersant du composant détergent permet d'améliorer la séparation des particules d'une suspension afin de prévenir l'agglutination, l'agrégation et/ou la décantation. Cet agent dispersant peut être un polymère soluble ou partiellement soluble dans l'eau tel que par exemple le polyéthylène glycol, les dérivés de la cellulose ou un polymère comprenant au moins un motif acide acrylique ou ester acrylique. Préférentiellement, l'agent dispersant est un polymère comprenant au moins un motif acide acrylique ou ester acrylique de formule générale -(CH₂-CH-COOR)- dans lequel R représente un groupe hydrogène, alkyle ou alkyle substitué, aryle ou aryle substitué. Par exemple, l'agent dispersant est un polymère ayant un poids moléculaire moyen Mw compris approximativement entre 500 et 10000.

Plus préférentiellement, l'agent dispersant est un polymère de l'acide acrylique. Par exemple, l'agent dispersant peut être un homopolymère de l'acide acrylique ayant un poids moléculaire moyen compris approximativement entre 2000 et 6000.

Par exemple, selon la présente invention, ledit agent dispersant dudit composant détergent est l'alkylglucoside en C₆.

La présence d'un agent dispersant dans la composition permet donc d'éviter toute agrégation de particules bactériennes lors du nettoyage de surfaces, ce qui assure une élimination optimale des particules de biofilms détachées d'un support sous l'action des enzymes. En effet, plutôt que de s'agréger, ces particules restent séparées dans une suspension, ne se redéposent pas et ne ré-adhèrent pas sur le support nettoyé.

L'agent mouillant du composant détergent est une substance chimique amphiphile, ou une composition comprenant ladite substance chimique amphiphile, qui modifie la tension superficielle entre deux surfaces. L'agent mouillant a pour avantage de favoriser l'étalement d'un liquide sur un solide mais aussi d'accentuer le contact entre deux surfaces. Plus particulièrement, l'agent mouillant favorise un contact entre le composant détergent et une surface et, par conséquent, entre les enzymes et leur substrat. Par exemple, sur des surfaces en inox fréquemment rencontrées dans les industries agro-alimentaires mais aussi en milieu hospitalier ou vétérinaire, l'agent mouillant permet de réaliser un étalement homogène de la composition et ainsi sa répartition parfaite sur les surfaces à décontaminer, par exemple sur les outils de production, les plans de travail, les sols ou encore les tables d'opérations et les outils médicaux.

L'agent mouillant peut être anionique, cationique, non-ionique ou zwitterionique. Préférentiellement, l'agent mouillant peut être un mouillant anionique ou non-ionique, c'est-à-dire que la partie hydrophile est chargée négativement ou ne comporte aucune charge nette, ou peut être une composition comprenant un mouillant anionique. Plus particulièrement, l'agent mouillant peut être un ester de saccharose ou une composition comprenant un alkyle sulfate de sodium et un alcool.

L'agent mouillant peut être moussant ou non moussant. De préférence, dans le composant détergent selon l'invention, ledit agent mouillant est non moussant à chaud et est de préférence choisi dans le groupe des sulfates d'alkyle sodique en C₆ à C₁₀, des sulfates d'alcool éthérés en C₆ à C₁₀ et des sulfonates d'alkylearyles en C₆ à C₁₀.

L'agent séquestrant est une substance chimique ayant la capacité à former des complexes avec des ions minéraux qu'il fixe sous une forme empêchant leur précipitation par les réactions habituelles. A titre d'exemple, l'agent séquestrant peut être l'acide éthylène-diamine-tétraacétique, le glucono-delta-lactone, le gluconate de sodium, le gluconate de potassium, le gluconate de calcium, l'acide citrique, l'acide phosphorique, l'acide tartrique, l'acétate de sodium, le sorbitol, un composé comportant un atome de phosphore. Préférentiellement, l'agent séquestrant peut être un oxyde de phosphore tel qu'un phosphonate, un phosphinate ou un phosphate ou leur mélange, ou un sel de celui-ci, une amine ou un oxyde d'amine portant au moins, dans sa structure, un groupement fonctionnel phosphine, oxyde de phosphine, phophinite, phosphonite, phosphite, phosphonate, phosphinate ou phosphate, seul ou en combinaison, ou un sel de ceux-ci.

Plus préférentiellement, l'agent séquestrant peut être un phosphonate ou un sel de celui-ci, une amine ou un oxyde d'amine comportant au moins, dans sa structure, un groupement fonctionnel phosphine, oxyde de phosphine, phosphinite, phosphonite, phosphite, phosphonate, phosphinate ou phosphate, seul ou en combinaison, ou un sel de ceux-ci. A titre d'exemple non limitatif, le phosphonate peut être de formule générale R¹(R²O)(R³O)P=O dans lequel R¹, R² et R³ représentent indépendamment un groupe hydrogène, alkyle, alkyle substitué, alkyle-amino substitué ou non, aminoalkyl substitué ou non, aryle ou aryle substitué. A titre d'exemple non limitatif, l'amine ou l'oxyde d'amine peuvent comporter un, deux ou trois substituant(s) de formule générale CR⁴R⁵W dans laquelle R⁴ et R⁵ représentent indépendamment l'un de l'autre un groupe hydrogène, alkyle, alkyle substitué, alkyle-amino substitué ou non, aminoalkyl substitué ou non, aryle ou aryle substitué, et W représente un groupe phosphonate, phosphinate ou phosphate. L'agent séquestrant peut être sous la forme d'un sel de sodium, calcium, lithium, magnésium ou potassium ; préférentiellement, l'agent séquestrant peut être sous la forme d'un sel de sodium, calcium ou potassium.

De préférence, l'agent séquestrant est un agent qui peut être utilisé sans danger dans le secteur alimentaire, c'est-à-dire que l'agent séquestrant ne présente aucun danger pour la santé, seul ou en association avec d'autres composants.

Avantageusement, selon l'invention, ledit au moins un composant enzymatique comprend au moins une enzyme additionnelle choisie dans le groupe constitué des a-polysaccharidoses (lactase, amylase, a-glucosidase, ...), des β-polysaccharidases (cellulase, hémi-cellulase, β-glucanase, arabanase, pectinase, chitinase, xylanase, dextranase, lysozyme, pullulanase, β-glucisidase, mannanase, ...), des oxydo-réductases (laccase, ...), des lyases (pectate lyase , ...), des transférases, des protéases et des peptidases (métallo-protéase, sérine-protéases, exo-peptidase, endo-protéase, cystine-protéase, ...), des lipases et des estérases (lysophospholipase, phospholipase, ...).

De préférence, la composition pour l'élimination de biofilms présente un pH compris entre 5 et 11.

Comme décrit ci-dessus, ledit au moins un composant détergent comprend une proportion d'agent séquestrant comprise entre 1 et 10%, une proportion d'agent dispersant comprise entre 1 et 10% et une proportion d'agent mouillant comprise entre 1 et 30% en poids par rapport au poids total du composant détergent. Préférentiellement, la proportion d'agent mouillant est comprise entre 5 et 20% en poids par rapport au poids total du composant détergent. De façon préférée, la proportion d'agent mouillant est de 15% en poids par rapport au poids total du composant détergent

Avantageusement, selon l'invention, ledit au moins un composant enzymatique et ledit au moins un composant détergent sont en solution dans un solvant, par exemple en solution dans un solvant de telle façon à former une solution vaporisable.

Préférentiellement, selon l'invention, ledit au moins un composant enzymatique et ledit au moins un composant détergent se présentent sous forme solide, par exemple sous forme d'un lyophilisat, d'une poudre, de granulés ou sous tout autre forme soluble dans un solvant.

Préférentiellement, ledit au moins un composant enzymatique est une solution dont le pH peut être compris approximativement entre 8 et 10.

Préférentiellement, ledit au moins un composant enzymatique est une solution aqueuse dont le pH peut être compris approximativement entre 5 et 11 ; plus préférentiellement le pH peut être approximativement entre 7 et 10, et ceci pour préserver au maximum l'intégrité des enzymes.

Alternativement, ledit au moins un composant enzymatique peut être sous forme solide tel que par exemple sous forme d'un lyophilisat, de poudres, de granulés ou sous tout autre forme permettant la solubilisation dudit composant dans un solvant, puis il sera ultérieurement dissous dans ledit solvant. Le solvant peut être de l'eau ou une solution aqueuse, acide, basique, alcoolique, tamponnée ou neutre. Ledit au moins un composant enzymatique solubilisé pourra alors dans ce cas être ultérieurement dilué dans une solution aqueuse contenant éventuellement un ou plusieurs composés tels que par exemple des détergents pour former la solution de nettoyage.

Comme pour ledit au moins un composant enzymatique, ledit au moins un composant détergent peut être sous forme solide à dissoudre dans un solvant et/ou dans une phase aqueuse ou sous forme liquide.

Lorsqu'il est sous forme solide, il peut soit être mis directement en solution dans la solution formée par le composant enzymatique éventuellement déjà dilué dans la phase aqueuse, soit être mis en solution dans un solvant, préalablement à sa dilution dans la solution formée par le composant enzymatique et la phase aqueuse, ou dans la phase aqueuse directement, avant la dilution du composant enzymatique.

Lorsque ledit au moins un composant détergent est sous forme liquide, les 100% du composant détergent sont éventuellement atteints généralement à l'aide d'eau et, préalablement à l'application sur le biofilm, il sera dilué dans une phase aqueuse, contenant éventuellement déjà le composant enzymatique.

D'autres formes de réalisation d'une composition suivant l'invention sont indiquées dans les revendications annexées.

L'invention a aussi pour objet un procédé d'élimination de biofilms présents sur une surface, ledit procédé comprenant les étapes suivantes :
a) mise à disposition
   - d'au moins un composant détergent contenant au moins un agent séquestrant et au moins un agent dispersant et au moins un agent mouillant dans les proportions décrites ci-dessus, et
   - d'au moins un composant enzymatique comprenant la β-1,6-N-acétylglucosaminidase ;
b) mise en solution ou dilution dudit au moins un composant détergent dans un solvant ;
c) mise en solution dudit au moins un composant enzymatique dans la solution formée à l'étape b) pour former une composition; ou
b') mise en solution ou dilution dudit au moins un composant enzymatique dans un solvant,
c') mise en solution dudit au moins un composant détergent dans la solution formée à l'étape b') pour former une composition,
d) application de ladite composition formée à l'étape c) ou c') sur ladite surface pendant une période de temps prédéterminée.

De préférence, selon le procédé suivant l'invention, ladite étape d'application de ladite la composition est associée à une étape d'abrasion mécanique de ladite surface avec ladite composition, par exemple par brossage mécanisé ou manuel ou encore par application à moyenne ou à haute pression. Une étape d'abrasion mécanique additionnelle permet à la solution comprenant ladite composition en phase aqueuse d'agir sur les différentes couches des biofilms mais aussi de participer mécaniquement à la déstructuration de la matrice polymère et ainsi ôter des pellicules de la surface des biofilms pour que l'enzyme et les autres composants de ladite composition atteignent encore mieux les différentes couches des biofilms, ce qui assure un traitement optimal des surfaces afin d'éliminer efficacement les biofilms.

Avantageusement, le procédé selon l'invention comprend une étape ultérieure d'application d'un biocide sur ladite surface. Un traitement biocide désinfectant additionnel, suite à l'action de la solution enzymatique lors de l'étape de traitement de surface par trempage, permet d'assurer la destruction des bactéries rendues libres en fin de traitement de ladite surface.

De préférence, selon le procédé suivant l'invention, ladite étape d'application de la solution de ladite la composition, est une étape ayant lieu pendant une période de temps prédéterminée comprise entre 1 minute et 1 heure, de préférence comprise entre 3 minutes et 30 minutes, d'une solution comprenant ladite composition et une phase aqueuse de dilution préalablement formée.

De préférence, le procédé suivant l'invention permet d'assurer une élimination d'au moins 75%, de préférence d'au moins 90% des microorganismes présents sur une surface et protégés par un biofilm.

Ce taux d'élimination des microorganismes, d'au moins 75%, de préférence d'au moins 90%, est calculé sur base des microorganismes présents dans un environnement donné (par exemple à la surface d'un outil médical ou à la surface d'un sol) avant et après une étape de traitement de surface suivant l'invention.

Par exemple, ce taux d'élimination des microorganismes peut être calculé par le biais d'une analyse luminométrique ATP-métrique qui permet de déterminer, par bioluminescence, la quantité de microorganismes présents sur une surface sur base des molécules d'Adénosine TriPhosphate qui y sont préalablement prélevées, par exemple par écouvillonnage. Cette analyse ATP-métrique donnant des résultats exprimés en unité relative de lumière (URL), le taux d'élimination des microorganismes est défini selon la formule suivante : 100 - [(valeur URL mesurée après traitement / valeur URL mesurée avant traitement) x 100].

Ce taux d'élimination des microorganismes peut également être établi par une analyse bactériologique consistant en un dénombrement des colonies bactériennes sur une même surface avant et après un traitement de surface réalisé suivant l'invention. Dans le cas d'analyses bactériologiques, le taux d'élimination des microorganismes est calculé selon la formule suivante : 100 - [(valeur CFU après traitement / valeur CFU avant traitement) x 100].

Il est bien entendu que toute autre technique bien connue de l'homme de métier et permettant de déterminer les quantités de microorganismes avant et après traitement d'une surface peut également être utilisée dans le cadre de la présente invention.

D'autres formes de réalisation du procédé suivant l'invention sont indiquées dans les revendications annexées.

D'autres formes d'utilisation d'une composition suivant l'invention sont indiquées dans les revendications annexées.

D'autres caractéristiques, détails et avantages de l'invention ressortiront de la description donnée ci-après, à titre non limitatif et en faisant référence aux figures annexées.
La figure 1 illustre les résultats obtenus en termes d'élimination du biofilm (réduction du biofilm %) formé par *Staphyloccocus epidermis* (EP = composition selon le document EP2243821 ; disp. = dispersant ; séq. = séquestrant ; mou. = mouillant ; β-1,6-N-= β-1,6-N-acétylglucosaminidase).
La figure 2 illustre les résultats obtenus en termes d'élimination du biofilm (réduction du biofilm %) formé par *Escherichia coli* (EP = composition selon le document EP2243821 ; disp. = dispersant; séq. = séquestrant ; mou. = mouillant ; β-1,6-N- = β-1,6-N-acétylglucosaminidase).
La figure 3 illustre les résultats obtenus en termes d'élimination du biofilm (réduction du biofilm %) formé par *Pseudomonas fluorescens* (EP = composition selon le document EP2243821 ; disp. = dispersant ; séq. = séquestrant ; mou. = mouillant ; β-1,6-N-= β-1,6-N-acétylglucosaminidase).
La figure 4 illustre les résultats des mesures d'activités enzymatiques de la β-1,6-N-acétylglucosaminidase au cours du temps selon que cette enzyme est formulée en présence de trois agents détergent (agent mouillant + agent dispersant + agent séquestrant) ou en présence de deux de ces trois agents détergent ou selon que cette enzyme n'est pas formulée dans un composant détergent.
La figure 5 illustre les résultats des mesures d'activités enzymatiques de trois cellulases au cours du temps selon que ces enzymes sont formulées ou non en présence de trois agents détergent (agent mouillant + agent dispersant + agent séquestrant) ou selon que cette enzyme n'est pas formulée dans un composant détergent.

### Exemples

### Exemple 1 - essais comparatifs : efficacité d'une composition utilisée dans un procédé selon l'invention et efficacité d'une composition selon le document EP2243821

Des essais comparatifs ont été réalisés afin de vérifier qu'une composition utilisée dans un procédé selon l'invention est au moins aussi efficace en termes d'élimination d'un biofilm qu'une composition telle que divulguée dans le document EP2243821 (cocktail de 3 enzymes dans un composant détergent comprenant 3 agents détergent).

La composition utilisée dans un procédé selon l'invention est la suivante :
- Composant enzymatique : β-1,6-N-acétylglucosaminidase à raison de 0,005% en volume par rapport au volume total de la composition ;
- Composant détergent : Biorem^{®}A1 à raison de 0,25% en volume par rapport au volume total de la composition.

La composition selon le document EP2243821 est la suivante :
- Composant enzymatique : Biorem^{®}10 à raison de 0,01% en volume par rapport au volume total de la composition ;
- Composant détergent : Biorem^{®}A1 à raison de 0,25% en volume par rapport au volume total de la composition.

Ces composants sont préparés à température ambiante par dilution de ceux-ci dans de l'eau avant de réaliser les essais comparatifs sur un biofilm développé par *Pseudomonas fluorescens*.

Les premières étapes du mode opératoire sont similaires aux étapes du protocole de l'annexe F de la Norme ISO 15883. En effet, le protocole de culture et de nettoyage du biofilm correspondent à ceux décrits dans cette Norme. La quantité de biofilms présente a quant à elle été évaluée à l'aide du kit de détection selon le document EP2243821.

Pratiquement, un biofilm a été développé par Pseudomonas fluorescens dans un tube de PTFE (PolyTretraFluoroEthylène) de diamètre intérieur de 6 mm. Le tube a été rempli d'une préculture liquide en phase de croissance exponentielle. La préculture a été préparée 24 h avant le test : un milieu de culture TSB a été inoculé avec la souche bactérienne *Pseudomonas fluorescens* à 30°C durant 12 h. Cette préculture a alors été diluée dans du milieu de culture TSB à 10% de manière à obtenir une densité optique comprise entre 300 nm et 600 nm. Le milieu de culture inoculé a alors été mis en circulation dans le tube en PTFE durant 3 jours. A la fin de cette période, le tube PTFE a été coupé en portions d'une taille comprise entre 5 et 10 cm sur lesquelles ont été testées les différentes compositions. Ces différentes compositions ont été mises en circulation durant 15 min à un débit de 100ml/min à une température de 45°C. Enfin, la quantité de biofilms présente a été évaluée par coloration de chaque portion de tube avec le kit de détection divulgué dans le document EP2537601.

Le kit de détection est composé de deux produits, un réactif de coloration et un réactif de nettoyage. Le colorant bleu présent dans le réactif de coloration adhère spécifiquement aux EPS (extracellular polymeric substances) formant la matrice protectrice du biofilm. La surface à analyser a été aspergée avec la solution de coloration. Après 5 min d'incubation sur la surface, l'excédent de la solution a été absorbée. Ensuite, la surface a été rincée à l'aide de la solution nettoyante du kit de détection qui requiert un temps de pose de 5 min. La surface a enfin été rincée à l'eau et a été analysée. La présence ou l'absence de biofilm a été démontrée par une couleur bleu plus ou moins foncée selon la quantité de biofilms existante sur la surface. Un coloration bleu intense indique la présence importante de biofilms.

Les résultats de ces essais sont présentés au tableau 1 ci-dessous.

**Tableau 1 :**

| **Nettoyage** | **Coloration** |
|---|---|
| Avant nettoyage | Coloration bleu intense |
| Eau | Coloration bleu intense |
| Composition selon le document EP2243821 | Absence de coloration bleu |
| Composition selon l'invention | Absence de coloration bleu |

Comme on peut le constater de ces essais comparatifs, pour des portions de tubes initialement fortement contaminées par du biofilm (coloration bleu intense avant nettoyage), un nettoyage à l'eau n'a eu aucun effet sur le biofilm et les portions de tubes initialement fortement contaminées par du biofilm ont conservé par conséquent leur coloration bleu intense.

Par contre, que ce soit dans le cas d'un nettoyage avec la composition selon le document EP2243821 ou dans le cas d'un nettoyage avec la composition utilisée dans le procédé selor l'invention, une élimination totale du biofilm a été assurée (absence de coloration bleu) après nettoyage avec ces deux compositions. Ceci indique donc qu'une composition utilisée dans le procédé selon l'invention permet d'éliminer des biofilms au moins de façon équivalente à la composition selon le document EP2243821 mais en ne mettant en œuvre qu'une seule enzyme, à savoir la β-1,6-N-acétylglucosaminidase, au lieu de trois enzymes.

### Exemple 2 - tests d'efficacité (en termes d'élimination du biofilm) d'une composition utilisée dans le procédé suivant l'invention selon que le composant détergent comprend simultanément un agent mouillant, un agent dispersant et un agent séquestrant ou une combinaison de deux de ces trois agents détergents

Des tests d'efficacité ont été réalisés sur du biofilm développé par différentes souches bactériennes, à savoir les souches *Staphyloccocus epidermis*, *Escherichia coli* et *Pseudomonas fluorescens*. Les biofilms ont été développés en laboratoire en conditions statiques dans des plaques multipuits et l'efficacité de la β-1,6-N-acétylglucosaminidase (12,5 ppm en enzyme active dans la composition, ce qui correspond à 0,00125% en volume par rapport au volume total de la composition) a été évaluée sur ces biofilms suite à sa formulation dans les composants détergent suivants :
- agent mouillant + agent séquestrant + agent dispersant = Biorem^{®}A1 à 1% en volume par rapport au volume total de la composition;
- agent mouillant du Biorem^{®}A1 à raison de 0,22% en volume par rapport au volume total de la composition + agent séquestrant du Biorem^{®}A1 à raison de 0,25% en volume par rapport au volume total de la composition;
- agent mouillant du Biorem^{®}A1 à raison de 0,22% en volume par rapport au volume total de la composition + agent dispersant du Biorem^{®}A1 à raison de 0,085% en volume par rapport au volume total de la composition ;
- agent séquestrant du Biorem^{®}A1 à raison de 0,25% en volume par rapport au volume total de la composition + agent dispersant du Biorem^{®}A1 à raison de 0,085% en volume par rapport au volume total de la composition.

En parallèle, la β-1,6-N-acétylglucosaminidase (12,5 ppm en enzyme active dans la composition, ce qui correspond à 0,00125% en volume par rapport au volume total de la composition) a été testée seule (non formulée dans un composant détergent mais en solution aqueuse) et l'efficacité d'une composition selon le document EP2243821 (BioremA1^{®} à 1% en volume par rapport au volume total de la composition et Biorem10^{®} à 0,025% en volume par rapport au volume total de la composition).

### A. Préparation des biofilms

Des colonies bactériennes ont été obtenues par ensemencements séparés de chacune des souches (*Staphyloccocus epidermis*, *Escherichia coli* ou *Pseudomonas fluorescens*) sur un milieu solide généraliste (PCA) incubé ensuite pendant 18 h à 37°C. Ensuite, quelques colonies bactériennes (environ 20 colonies) ont été suspendues dans leur milieu de culture adéquat respectif. Plus particulièrement, les colonies bactériennes ont été suspendues dans les milieux de cultures suivants :
- *Staphyloccocus epidermis* : suspension d'environ 20 colonies dans 2 mL de TGN (30 g/L de Tryptic Soy Broth + 1 g/L de glucose + 2 g/L de NaCl) ;
- *Escherichia coli* : suspension d'environ 20 colonies dans 2 mL de LBG (15 g/L d'eau peptonée + 5 g/L d'extrait de levure + 10 g/L de glucose) ;
- *Pseudomonas fluorescens* : suspension d'environ 20 colonies dans 2 mL de TSB (30 g/L de Tryptic Soy Broth).

Chacune des suspensions a été ajustée par ajout de milieu de culture (TGN ou LBG ou TSB) de telle sorte à mesurer une densité optique (D.O.) de 0,05 à 620 nm puis les suspensions bactériennes ont été placées dans les puits de plaques multipuits à raison de 100µL par puits pour *Staphyloccocus epidermis*, 220 µL par puits pour Escherichia coli et 220 µL par puits pour *Pseudomonas fluorescens* avant incubation durant 24 h à 37°C pour *Staphyloccocus epidermis*, durant 48 h à 30°C pour *Escherichia coli* et durant 48 h à 30°C pour *Pseudomonas fluorescens*. Bien entendu, pour chacune des souches bactériennes testées, des puits ont été rempli uniquement avec du milieu de culture afin de constituer des contrôles négatifs.

### B. Traitement enzymatique des biofilms formés et quantification de l'élimination des biofilms

Avant de traiter les biofilms formés avec diverses compositions dont une composition utilisée dans le procédé selon l'invention, les puits des plaques multi-puits ont été vidés des milieux de culture ayant permis l'établissement et le développement des différents biofilms. Le traitement des biofilms en lui-même a été réalisé par remplissage des puits avec 1 mL des solutions (compositions) de nettoyage, de l'eau adoucie étant uniquement ajoutée dans les deux puits du témoin positif, les 2 puits du témoin négatif restant vides quant à eux. Suite à ces remplissages, une incubation à 45°C pendant 15 min a été respectée (durée de nettoyage pour éliminer les biofilms).

Après ce temps d'incubation, les puits ont été vidés des solutions de nettoyage et ont été rincés à l'aide d'une solution saline stérile à 0,9% avant séchage des plaques pendant 1 h à 60°C. Suite à ce séchage, 0,5 mL de cristal violet à 0,1% ont été ajoutés dans chaque puits avant incubation durant 15 min à température ambiante. Suite aux 15 min d'incubation à température ambiante, les puits ont été vidés de la solution de cristal violet, rincés deux fois avec de l'eau distillée puis séchés sur du papier absorbant si nécessaire. 1mL d'acide acétique à 33 % a été ajouté ensuite dans chaque puits avant incubation durant 1 h à température ambiante avant la réalisation d'une lecture des plaques à la longueur d'onde de 570 nm (mesure d'absorbance par spectrophotométrie - Thermo Spectronic UV1). La réduction de la quantité de biofilm est calculée par rapport à un témoin non nettoyé et à un témoin ne contenant pas de biofilm: le cristal violet (CV) est un colorant cationique qui va se lier de façon aspécifique aux constituants du biofilm chargés négativement et les marquer en bleu. Les résultats de mesure d'absorbance du cristal violet permettent donc de quantifier le biofilm encore présent après nettoyage.

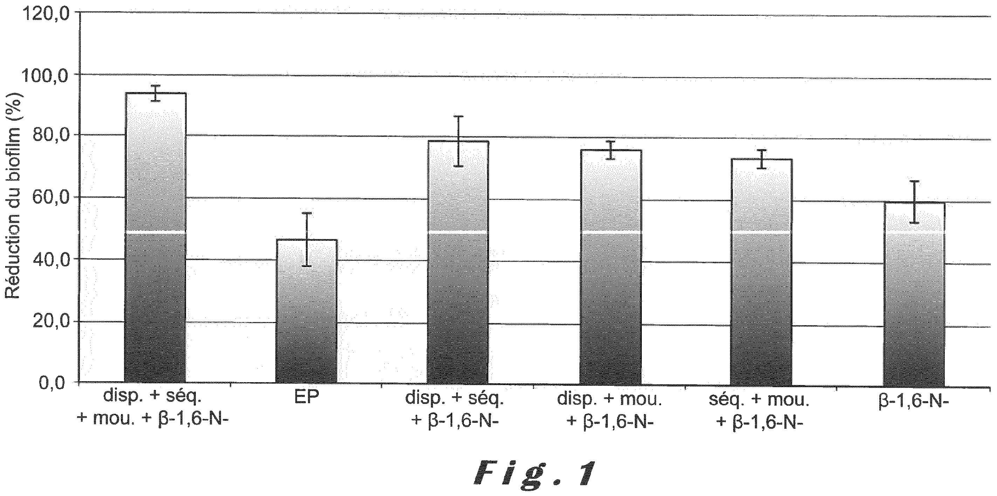

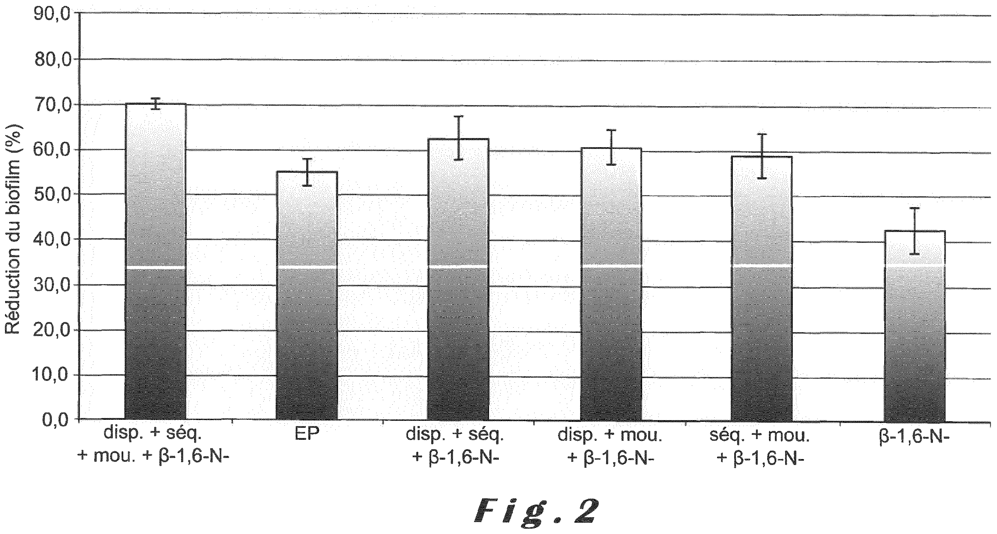

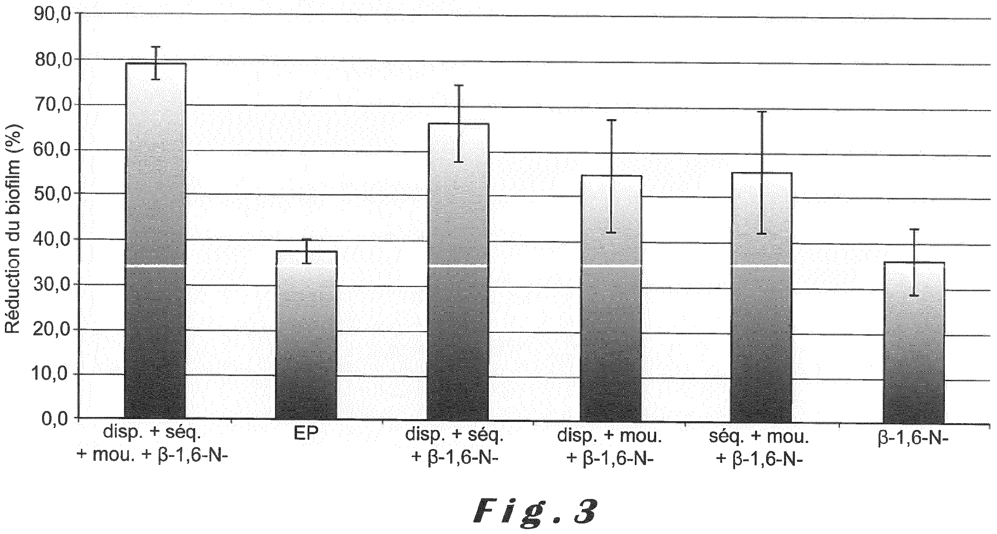

Les résultats obtenus sont présentés aux figures 1, 2 et 3, respectivement pour les biofilms formés par *Staphyloccocus epidermis, Escherichia coli* ou *Pseudomonas fluorescens.*

Comme on peut le constater, quel que soit le biofilm considéré (formé par *S*. *epidermis* ou par *E. coli* ou par *P*. *fluorescens*), une composition selon l'invention (β-1,6-N- + disp. + séq. + mou.) permet d'éliminer de façon significativement supérieure les biofilms par rapports aux autres compositions testées. Ceci démontre que les trois agents détergents présentent un effet synergique inattendu permettant d'optimiser l'action de la β-1,6-N-acétylglucosaminidase en termes d'élimination des biofilms. En effet, lorsque la β-1,6-N-acétylglucosaminidase est formulée dans un composant détergent ne comprenant que deux des trois agents détergent, il est observé que l'élimination du biofilm est moindre. La même constatation peut être faite avec une composition selon le document EP2243821 qui ne permet pas d'éliminer aussi efficacement les biofilms en comparaison avec une composition utilisée dans le procédé selon l'invention.

### Exemple 3 - activité enzymatique de la β-1,6-N-acétylglucosaminidase selon que le composant détergent comprend simultanément un agent mouillant, un agent dispersant et un agent séquestrant ou une combinaison de deux de ces trois agents détergents

L'activité enzyme au cours du temps de la_β-1,6-N-acétylglucosaminidase a été mesurée à l'aide du substrat 4-nitrophenyl-N-acetyl-b-D-glucosaminide. Ce substrat libère un composé jaune lorsqu'il est hydrolysé par la β-1,6-N-acétylglucosaminidase. Plus le taux d'hydrolyse est important plus la concentration en composé jaune est élevée et plus la β-1,6-N-acétylglucosaminidase est active. Le taux d'hydrolyse est quantifié par mesure de l'absorbance de la solution à 405 nm et permet de juger de l'activité enzymatique de la β-1,6-N-acétylglucosaminidase.

De façon générale, l'activité enzymatique au cours du temps de la β-1,6-N-acétylglucosaminidase a été mesurée selon les étapes suivantes :
a) la β-1,6-N-acétylglucosaminidase a été mélangées dans les différentes compositions à tester (voir ci-dessous) et les solutions ainsi obtenues ont été maintenues à une température de 40°C durant 2 h afin de simuler les conditions de nettoyage;
b) des échantillons de ces solutions ont été prélevés à t = 0 (t₀) et t = 2 h (t₂ₕ) afin de mesurer l'activité enzymatique au cours du temps de la β-1,6-N-acétylglucosaminidase.

Plus particulièrement, le protocole suivant a été suivi :
a) une solution tamponnée à pH 5,9 a été préparée : phosphate de sodium 50 mM + 4-nitrophenyl-N-acetyl-b-D-glucosaminide 5 mM + NaCl 100 mM ;
b) la solution obtenue en a) a été préchauffée à 37°C et répartie à raison de 1mL dans des Eppendorts^{®};
c) 100 µL des compositions dont l'activité enzymatique au cours du temps doit être mesurée (dont une composition selon l'invention) ont été placés dans les Eppendorfs^{®} du point b) ;
d) la concentration en β-1,6-N-acétylglucosaminidase a été ajustée par dilution avec du tampon phosphate si nécessaire afin d'obtenir une concentration d'environ 3,7 µg/mL en β-1,6-N-acétylglucosaminidase ;
e) la réaction a été stoppée par ajout de 5 µL de NaOH 10N après 5 min ;
f) l'absorbance a été mesurée à 405 nm (- Thermo Spectronic UV1 après 5 minutes (à t₀ + 5 min et t₂ₕ + 5 min), un contrôle négatif (blanc) étant préparé uniquement avec de l'eau.

Plus particulièrement, l'activité enzymatique de la_β-1,6-N-acétylglucosaminidase a été mesurée au cours du temps dans les compositions suivantes :
- agent mouillant + agent séquestrant + agent dispersant (= Biorem^{®}A1 à 0,25% en volume par rapport au volume total de la composition) + β-1,6-N-acétylglucosaminidase à 7,4 µg/mL;
- agent mouillant du Biorem^{®}A1 à raison de 0,055% en volume par rapport au volume total de la composition + agent séquestrant du Biorem^{®}A1 à raison de 0,0625% en volume par rapport au volume total de la composition + β-1,6-N-acétylglucosaminidase à 7,4 µg/mL;
- agent mouillant du Biorem^{®}A1 à raison de 0,055% en volume par rapport au volume total de la composition + agent dispersant du Biorem^{®}A1 à raison de 0,021% en volume par rapport au volume total de la composition + β-1,6-N-acétylglucosaminidase à 7,4 µg/mL;
- agent séquestrant du Biorem^{®}A1 à raison de 0,0625% en volume par rapport au volume total de la composition + agent dispersant du Biorem^{®}A1 à raison de 0,021% en volume par rapport au volume total de la composition + β-1,6-N-acétylglucosaminidase à 7,4 µg/mL;
- β-1,6-N-acétylglucosaminidase seule à 7,4 µg/mL en solution aqueuse.

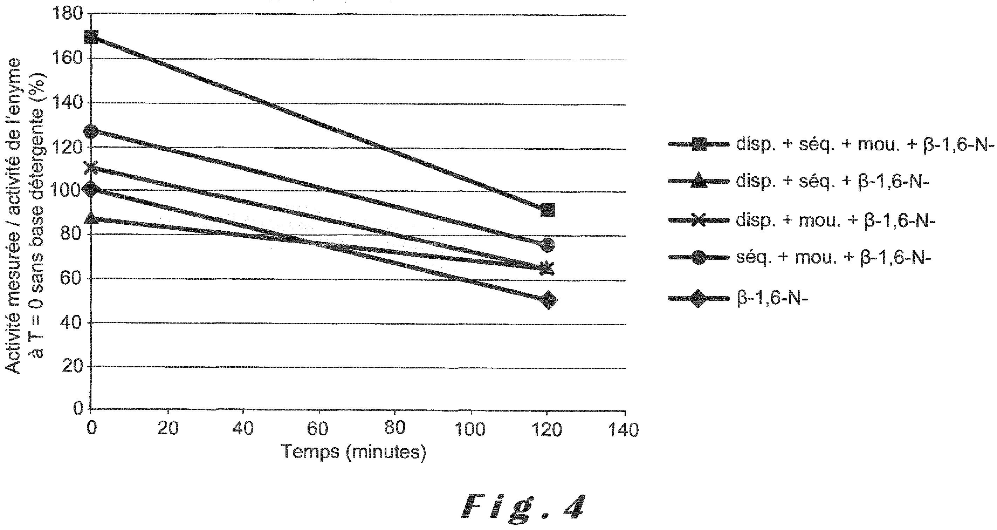

Les résultats obtenus sont illustrés à la figure 4 et sont exprimés en % de l'activité_B-1,6-N-acétylglucosaminidase mesurée pour une composition ne comprenant pas de composant détergent (activité enzymatique relative). Comme on peut le constater, dès le départ à t₀, c'est-à-dire dès le moment où la β-1,6-N-acétylglucosaminidase est formulée dans un composant détergent comprenant simultanément un agent mouillant, un agent séquestrant et un agent dispersant, son activité enzymatique est fortement et significativement potentialisée/augmentée par rapport aux activités enzymatiques mesurées pour la β-1,6-N-acétylglucosaminidase lorsqu'elle est formulée dans un composant détergent ne comprenant que deux des trois agents détergent. Par la suite, au cours du temps et en particulier après 2 h, une activité enzymatique de la β-1,6-N-acétylglucosaminidase significativement supérieure est observée pour une composition utilisée dans le procédé selon l'invention par rapport aux activités enzymatiques mesurées pour la β-1,6-N-acétylglucosaminidase lorsqu'elle est formulée dans un composant détergent ne comprenant que deux des trois agents détergent.

Ceci démontre bien l'effet synergique des trois agents détergent qui, ensemble, potentialisent/augmentent de façon significative l'activité enzymatique de la β-1,6-N-acétylglucosaminidase, laquelle est par conséquent d'autant plus efficace au cours du temps.

A titre de comparaison avec la β-1,6-N-acétylglucosaminidase, trois cellulases (autres enzymes d'une autre famille - Celluclast^{®} 1.5L, Carezyme^{®} 4500L, Viscozyme^{®} 120L) ont également été testées en termes d'activité enzymatique au cours du temps après formulation dans du Biorem^{®}A1 comme composant détergent à raison de 0,025% en volume par rapport au volume total de la composition. En particulier, l'activité enzymatique des trois cellulases a été mesurée selon le protocole endo-cellulase (méthode CELLG5^{®}) du kit Megazyme^{®} en respectant le protocole recommandé par le fabriquant. Les activités enzymatiques des cellulase seules ou des cellulases formulées dans un composant détergent selon l'invention ont ainsi été comparées.

La quantité de cellulase dans la composition a été établie de telle sorte à être dans une gamme de concentration qui soit dans la gamme de mesure du kit commercial. Des tests préliminaires ont été réalisés pour chacune des cellulase testée et une concentration adéquate pour la mesure a été sélectionnée pour chaque cellulase.

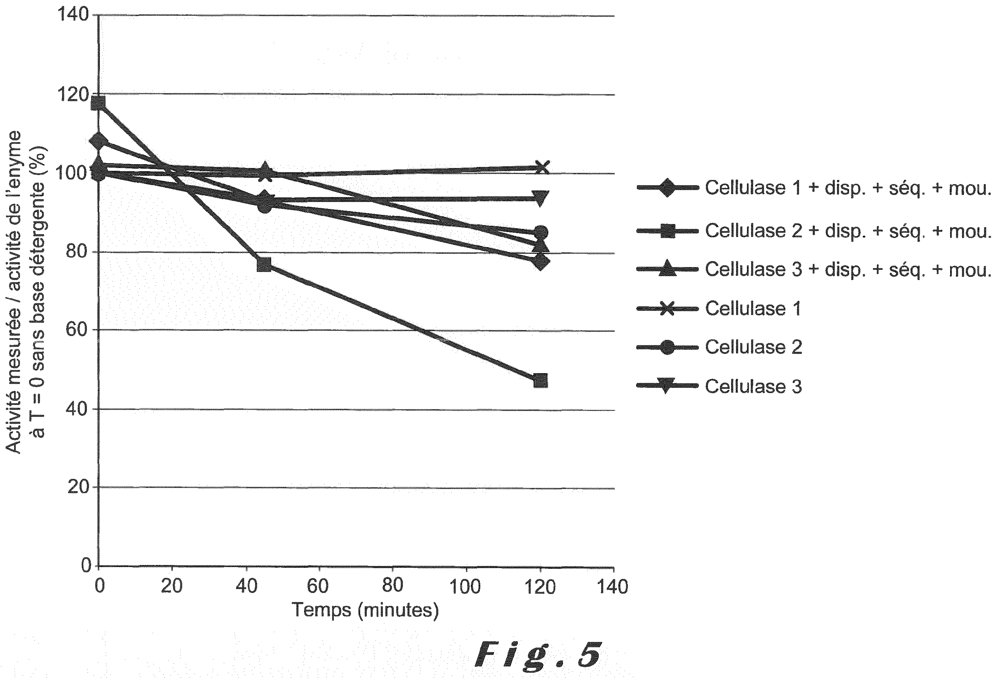

La figure 5 illustre les résultats obtenus et on peut constater que, contrairement aux résultats obtenus pour la β-1,6-N-acétylglucosaminidase, le même composant détergent (agent mouillant + agent dispersant + agent séquestrant) n'a absolument pas le même effet sur les cellulases. En effet, aucune potentialisation/augmentation de l'activité enzymatique n'est observée directement suite à la formulation de chacune des cellulases dans ce composant détergent. En outre, pour ces trois cellulases, aucune potentialisation/augmentation de l'activité enzymatique n'est maintenue au cours du temps par ce composant détergent. Que du contraire, pour la Cellulase 2, sa formulation dans un composant détergent identique à celui d'une composition utilisée dans le procédé selon l'invention en diminue très fortement l'activité enzymatique. Ceci met d'autant plus en avant l'effet surprenant observé avec une composition utilisée dans le procédé selon l'invention où la β-1,6-N-acétylglucosaminidase, au contraire de ces trois cellulases, a une activité enzymatique significativement augmentée lorsqu'elle est en présence des trois agents détergent (agent mouillant + agent dispersant + agent séquestrant).

Il est bien entendu que la présente invention n'est en aucune façon limitée aux formes de réalisations décrites ci-dessus et que bien des modifications peuvent y être apportées sans sortir du cadre des revendications annexées.

## Revendications

1. Procédé d'élimination de biofilms présents sur une surface, ledit procédé comprenant les étapes suivantes :
a) mise à disposition
- d'au moins un composant détergent contenant au moins un agent séquestrant et au moins un agent dispersant et au moins un agent mouillant, et
- d'au moins un composant enzymatique comprenant la β1,6--N-acétylglucosaminidase ;
b) mise en solution ou dilution dudit au moins un composant détergent dans un solvant ;
c) mise en solution dudit au moins composant enzymatique dans la solution formée à l'étape b) pour former une composition dans laquelle la proportion d'agent séquestrant est comprise entre 1 et 10%, la proportion en agent dispersant est comprise entre 1 et 10% et la proportion d'agent mouillant est comprise entre 1 et 30% en poids par rapport au poids total du composant détergent ;
ou
b') mise en solution ou dilution dudit au moins un composant enzymatique dans un solvant,
c') mise en solution dudit au moins un composant détergent dans la solution formée à l'étape b') pour former une composition dans laquelle la proportion d'agent séquestrant est comprise entre 1 et 10%, la proportion en agent dispersant est comprise entre 1 et 10% et la proportion d'agent mouillant est comprise entre 1 et 30% en poids par rapport au poids total du composant détergent,
d) application de ladite composition formée à l'étape c) ou c') sur ladite surface pendant une période de temps prédéterminée.

2. Procédé selon la revendication 1, **caractérisé en ce que** ladite étape d'application de ladite composition est associée à une étape d'abrasion mécanique de ladite surface avec ladite composition, par exemple par brossage mécanisé ou manuel ou encore par application à moyenne ou à haute pression.

3. Procédé selon la revendication 1 ou 2, **caractérisé en ce qu'**il comprend une étape ultérieure d'application d'un biocide sur ladite surface.

4. Procédé selon une quelconque des revendications précédentes dans lequel le composant enzymatique comprend au moins une enzyme additionnelle choisie dans le groupe constitué des α-polysaccharidases, des β-polysaccharidases, des oxydo-réductases, des lyases, des transférases, des protéases et des peptidases des lipases et des estérases.

5. Utilisation d'une composition comprenant au moins un composant enzymatique comprenant la β-1,6-N-acétylglucosaminidase et au moins un composant détergent contenant au moins un agent mouillant, au moins un agent séquestrant et au moins un agent dispersant, pour l'élimination de biofilms présents sur une surface par nettoyage en place, par trempage ou par vaporisation de ladite surface, dans laquelle la proportion dans ledit composant détergent d'agent séquestrant est comprise entre 1 et 10%, la proportion en agent dispersant est comprise entre 1 et 10% et la proportion d'agent mouillant est comprise entre 1 et 30% en poids par rapport au poids total du composant détergent.

## Patentansprüche

1. Verfahren zum Entfernen von Biofilmen, die auf einer Oberfläche vorhanden sind, das Verfahren umfassend die folgenden Schritte:
a) Bereitstellen
- mindestens einer Reinigungsmittelkomponente, die mindestens ein Sequestriermittel und mindestens ein Dispergiermittel und mindestens ein Netzmittel enthält, und
- mindestens einer Enzymkomponente, umfassend β1,6--N-Acetylglucosaminidase;
b) Auflösen oder Verdünnen der mindestens einen Reinigungsmittelkomponente in einem Lösungsmittel;
c) Auflösen der mindestens einen Enzymkomponente in der in Schritt b) gebildeten Lösung, um eine Zusammensetzung zu bilden, in der der Anteil an Sequestriermittel zwischen 1 und 10 % liegt, der Anteil an Dispergiermittel zwischen 1 und 10 % liegt und der Anteil an Netzmittel zwischen 1 und 30 Gewichts-% liegt, bezogen auf das Gesamtgewicht der Reinigungsmittelkomponente;
oder
b') Auflösen oder Verdünnen der mindestens einen Enzymkomponente in einem Lösungsmittel,
c') Auflösen der mindestens einen Reinigungsmittelkomponente in der in Schritt b') gebildeten Lösung, um eine Zusammensetzung zu bilden, in der der Anteil an Sequestriermittel zwischen 1 und 10 Gewichts-% liegt, der Anteil an Dispergiermittel zwischen 1 und 10 Gewichts-% liegt und der Anteil an Netzmittel zwischen 1 und 30 Gewichts-% liegt, bezogen auf das Gesamtgewicht der Reinigungsmittelkomponente,
d) Aufbringen der in Schritt c) oder c') gebildeten Zusammensetzung auf die Oberfläche über eine vorbestimmte Zeitdauer.

2. Verfahren nach Anspruch 1, **dadurch gekennzeichnet, dass** der Schritt eines Aufbringens der Zusammensetzung mit einem Schritt mechanischer Abrasion der Oberfläche mit der Zusammensetzung assoziiert ist, beispielsweise durch mechanisiertes oder manuelles Bürsten oder durch Aufbringen bei mittlerem oder hohem Druck.

3. Verfahren nach Anspruch 1 oder 2, **dadurch gekennzeichnet, dass** es einen nachfolgenden Schritt eines Aufbringens eines Biozids auf die Oberfläche umfasst.

4. Verfahren nach einem der vorherigen Ansprüche, wobei die Enzymkomponente mindestens ein zusätzliches Enzym umfasst, das ausgewählt ist aus der Gruppe, bestehend aus α-Polysaccharidasen, β-Polysaccharidasen, Oxidoreduktasen, Lyasen, Transferasen, Proteasen und Peptidasen von Lipasen und Esterasen.

5. Verwendung einer Zusammensetzung, umfassend mindestens eine Enzymkomponente, umfassend β-1,6-N-Acetylglucosaminidase, und mindestens eine Reinigungsmittelkomponente, die mindestens ein Netzmittel, mindestens ein Sequestriermittel und mindestens ein Dispergiermittel enthält, zum Entfernen von Biofilmen, die auf einer Oberfläche vorhanden sind, durch Reinigung vor Ort, durch Einweichen oder Besprühen der Oberfläche, wobei der Anteil an Sequestriermittel zwischen 1 und 10 Gewichts-% liegt, der Anteil an Dispergiermittel zwischen 1 und 10 Gewichts-% liegt und der Anteil an Netzmittel zwischen 1 und 30 Gewichts-% liegt, bezogen auf das Gesamtgewicht der Reinigungsmittelkomponente.

## Claims

1. Method for removing biofilms present on a surface, said method comprising the following steps:
a) providing
- at least one detergent component containing at least one sequestering agent, at least one dispersing agent, and at least one wetting agent, and
- at least one enzymatic component comprising β1,6-N-acetylglucosaminidase;
b) dissolving or diluting said at least one detergent component in a solvent;
c) dissolving said at least one enzymatic component in the solution formed in step (b) to form a composition in which the proportion of sequestering agent is between 1 and 10%, the proportion of dispersing agent is between 1 and 10%, and the proportion of wetting agent is between 1 and 30% by weight relative to the total weight of the detergent component;
or
b') dissolving or diluting said at least one enzymatic component in a solvent,
c') dissolving said at least one detergent component in the solution formed in step b') to form a composition in which the proportion of sequestering agent is between 1 and 10%, the proportion of dispersing agent is between 1 and 10%, and the proportion of wetting agent is between 1 and 30% by weight relative to the total weight of the detergent component,
d) applying said composition formed in step c) or c') to said surface for a predetermined period of time.

2. Method according to claim 1, **characterised in that** said step of applying said composition is associated with a step of mechanically abrading said surface using said composition, for example by mechanised or manual brushing, or alternatively by medium- or high-pressure application.

3. Method according to claim 1 or 2, **characterised in that** it comprises a subsequent step of applying a biocide to said surface.

4. Method according to any one of the preceding claims, wherein the enzymatic component comprises at least one additional enzyme selected from the group consisting of α-polysaccharidases, β-polysaccharidases, oxidoreductases, lyases, transferases, proteases, peptidases, lipases, and esterases.

5. Use of a composition comprising at least one enzymatic component comprising β-1,6-N-acetylglucosaminidase and at least one detergent component containing at least one wetting agent, at least one sequestering agent and at least one dispersing agent, for the removal of biofilms present on a surface by clean-in-place cleaning, soaking or spraying of said surface, wherein the proportion of sequestering agent in said detergent component is between 1 and 10%, the proportion of dispersing agent is between 1 and 10%, and the proportion of wetting agent is between 1 and 30% by weight relative to the total weight of the detergent component.
